(19) Europäisches Patentamt
European Patent Office
Office européen des brevets

(11) Publication number: **0 286 131 B1**

# (12) EUROPEAN PATENT SPECIFICATION

(45) Date of publication of patent specification: **02.09.92**

(51) Int. Cl.5: **C07C 19/08**, C07C 17/33

(21) Application number: **88105654.3**

(22) Date of filing: **08.04.88**

## (54) Perfluoro-2,3,4-trimethylpentane and process for the preparation thereof.

(30) Priority: **10.04.87 IT 2006087**

(43) Date of publication of application:
**12.10.88 Bulletin 88/41**

(45) Publication of the grant of the patent:
**02.09.92 Bulletin 92/36**

(84) Designated Contracting States:
**DE ES FR GB NL SE**

(56) References cited:
**EP-A- 0 196 630**
**WO-A-88/08007**
**DE-A- 2 302 132**
**DE-A- 2 332 097**

(73) Proprietor: **AUSIMONT S.p.A.**
**Foro Buonaparte, 31**
**I-20121 Milano(IT)**

(72) Inventor: **Tonelli, Claudio**
**25, via Gino Valagussa**
**I-20049 Concorezzo Milan(IT)**
Inventor: **Tortelli, Vito**
**46, viale Corsica**
**I-20137 Milan(IT)**

(74) Representative: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

Perfluoroalkanes, thanks to their non-toxicity, uninflammability, chemical and thermal resistance and unusual electric properties, find increasing use in many technologically advanced industrial sectors.

There are several known processes for preparing them. Perfluoroalkanes can for example be obtained by fluorination with $COF_3$ of mainly aromatic hydrocarbons; E.J. Barber, J. Burdon, I.W. Parson, J.C. Tatlow Tetrahedron vol. 28, pages 43-52 (1972).

Perfluoroalkanes are otherwise prepared by direct fluorination of aliphatic hydrocarbons with elemental fluorine (R.J. Lagow, J.L. Margreve Progr. Inorg. Chem. Vol. 26, pages 161-210 (1979).

Both of these processes are not selective because, besides giving rise to the desired perfluoroalkanes, they also give rise to by-product mixtures containing partially hydrogenated fluoroalkanes or perfluoroalkanes having a lower number of carbon atoms, and do so in an uncontrolled way. Fluorination leads in fact to the easy rupture of simple C-C- bonds. This, of course, limits the industrial utilization of the latter processes.

Additionally, the electrochemical technique for fluorinating hydrogenated substrates is of little use in the fluorination of hydrocarbons; in fact, it is essentially used for the fluorination of carboxylic and sulphonic acids.

US-A-3962358 describes a process for fluorinating perfluoroolefins with elemental fluorine to obtain corresponding perfluoroalkanes with good yields. This process, however, permits perfluoroalkanes to be obtained with 6 and 9 carbon atoms with respective boiling points of 60 and 130 ° C. It is not possible to synthesize perfluoroalkanes with intermediate boiling points.

Conversely, some electronic appliances do need to have fluids available which have boiling points ranging from 100 to 130 ° C, as these can advantageously be used as cooling fluids to cool laser and electronic circuits in general, or as test fluids in electronics as for example in the Gross Leak test by the NID method in accordance with MIL STD 883C (August 25, 1983), procedure F.

Fluids available on the market are the fluorohydrocarbon fluids for electronics having boiling points up to 102 ° C, such as for example FC® 75 and 77, or fluorohydrocarbon fluids with boiling points higher than 170 ° C (FC® 43, boiling point = 175 ° C; and FC® 70, boiling point = 215 ° C); there are however, no fluids available with intermediate boiling points, nor is it advisable to use a mixture of the above-cited products in order to obtain fluids with boiling points in the abovementioned range.

The object of the present invention is therefore, to provide a new perfluoroalkane by a more selective process compared to the ones in the art.

Another object of the present invention is to obtain a thermally stable perfluoroalkane, which can be utilized in the electronic industry both as a cooling fluid and as fluid for testing electronic circuits.

The above objects have been achieved by obtaining a new fluorinated compound, i.e. perfluoro-2,3,4-trimethylpentane of formula:

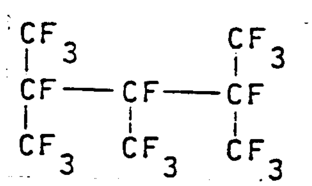

(I)

prepared by subjecting to fluorination with elemental fluorine, in the presence of UV radiation and at temperatures comprised between -40 and + 40 ° C the hexafluoropropene trimer of formula:

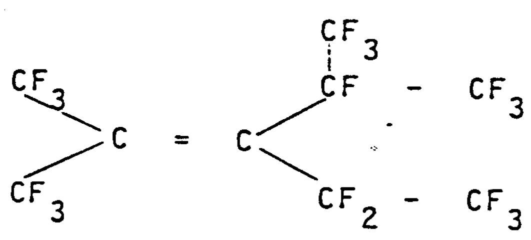

(II)

eventually in the presence of the olefin of formula (III)

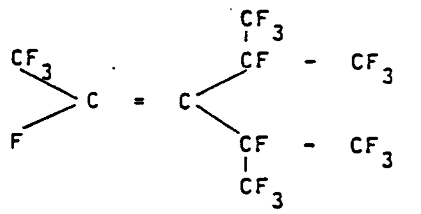

(III)

The trimers of formula (II) and (III) were obtained according to the process as described in US-A-3917724.

The reaction is preferably conducted at temperatures ranging from 10 to 35 ° C; UV-radiation is obtained by means of a high pressure mercury discharge lamp.

Generally, fluorination is conducted in the presence or absence of an inert gas as a diluent.

The reaction can be conveniently carried out in the presence of a perfluorinated inert solvent preferably a perfluoropolyether having a molecular weight comprised in the range of from 800 to 2000 and selected from one of the following classes:

(a) $CF_3O\ (C_2F_4O)_p\ (CF_2O)_q\text{-}CF_3$
wherein p and q are integers and the p/q ratio ranges from 1 to 0.5, the units $C_2F_4O$ and $CF_2O$ being randomly distributed along the chain;

(b) $RfO(C_3F_6O)_m\ (CFXO)_n\ Rf$
wherein Rf is $CF_3$, $C_2F_5$ or $C_3F_7$, X is either F or $CF_3$, m and n are integers chosen so as to fulfil the conditions that the molecular weight ranges from 800 to 2000;

(c) $C_3F_7O\ (C_3F_6O)_x\ C_2F_5$ wherein x is an integer chosen so as to fulfil the condition that the molecular weight ranges from 800 to 2000;

(d) $R'f\ (CF_2CF_2O)_n\ R'f$
wherein n is an integer chosen so as to fulfil the above mentioned condition and R'f is $CF_3$ or $C_2F_5$;

(e) $A(CF_2CF_2CF_2O)_n\ B$
wherein n is an integer chosen so as to fulfil the above condition , A is F or OR'f and B is R'f or $C_3F_7$;

(f) perfluoropolyethers containing repeating units $(C_2F_4O)$,$(C_3F_6O)$, (CFXO) and having a molecular weight of from 800 to 2000;

(g)

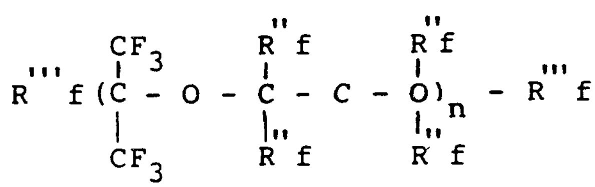

wherein R‴ f is a perfluoroalkyl group, n is an integer chosen so as to fulfil the above condition and R″f is F or a perfluoroalkyl group.

The perfluoropentane of the present invention is obtained in the presence of new highly branched perfluoroalkanes, object of Italian Patent Application No. 20061 A/87, corresponding to PCT application (Publication Number WO 88/08007) filed concurrently with the present application.

The products according to the present invention can easily be separated from the other reaction products with good yields by rectification with high purity degree.

Furthermore, as it has a boiling point of 115 ° C, it is highly suited to be used for cooling laser circuits such as those of aeronautic systems and for the cited tests in electronics.

The following examples are given for merely illustrative purposes and are not to be construed as a limitation of the present invention.

## EXAMPLE 1

Into a quartz reactor having an optical path of 5 mm and a volume of 2000 ml, 300 g (0.76 moles) of product (II) as defined above were charged and after generation of a $N_2$ atmosphere in the reactor, a flow of $F_2$ and $N_2$ (1:1) equal to 2 l/h was introduced. While maintaining the inner temperature at 33 ° C, irradiation was simultaneously carried out using a high pressure mercury discharge lamp type Hanau® TQ 150 (wave length ranging from 254 to 400 nanometers).

The reaction trend was followed by gas-chromatographic analysis. After 24 hours, the starting olefin was no longer present. $F_2$ flow and UV-irradiation were stopped and 310 g of a colorless fluid having a boiling point of 115 °C at 760 mm of Hg and the following structure ($^{19}F$-NMR, $\delta$, $CFCl_3$):

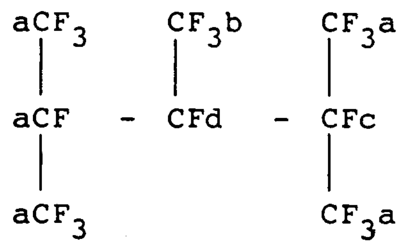

a = 70 ppm
b = 68 ppm
c,d 169-170 ppm

A fraction essentially consisting of perfluoroalkanes having 9 carbon atoms remained in the vessel.

EXAMPLE 2

150 g of a mixture containing 94% by weight of the perfluoroolefin of formula II and 6% by weight of the perfluoroolefin of formula III and 150 g of a perfluoropolyether having a molecular weight of 1800 and belonging to class (b) were loaded in the reactor according to example 1 while maintaining the inner temperature at 15 °C. Irradiation was simultaneously carried out using the UV lamp of Example 1.

At the end of the reaction 135 g of perfluorinated products were removed from the solvent by means of distillation and subsequently by rectification 56 g of product of formula (I) were isolated (yield 41%).

## Claims

**1.** Perfluoro-2,3,4-trimethylpentane.

**2.** A process for preparing the compound according to claim 1, wherein the compound of formula (II):

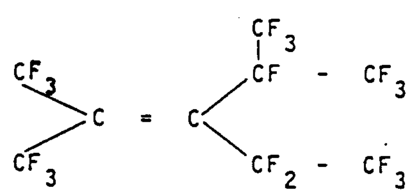

(II)

optionally in the presence of a compound of formula (III)

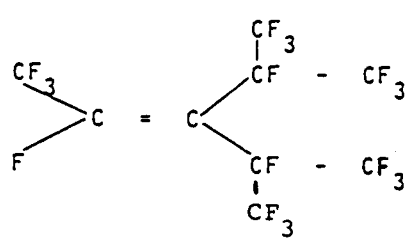

(III)

is subjected to fluorination with elemental fluorine in the presence of UV-radiation at temperatures higher than -40 °C but not exceeding 40 °C.

**3.** The process according to claim 2, wherein the temperature ranges from 10 to 35 ° C.

**4.** The process according to claim 2 carried out in the presence of a fluorinated inert solvent wherein the solvent is a perfluoropolyether having a molecular weight in the range of from 800 to 2000 and being selected from one of the following classes:
(a) $CF_3O\ (C_2F_4O)_p\ (CF_2O)_q\ \text{-}CF_3$
wherein p and q are integers and the p/q ratio ranges from 1 to 0.5, the units $C_2F_4O$ and $CF_2O$ being randomly distributed along the chain;
(b) $RfO(C_3F_6O)_m\ (CFXO)_n\ Rf$
wherein Rf is $CF_3$,$C_2F_5$ or $C_3F_7$, X is F or $CF_3$, m and n are integers chosen so as to fulfil the conditions that the molecular weight ranges from 800 to 2000;
(c) $C_3F_7O\ (C_3F_6O)_x\ C_2F_5$ wherein x is an integer chosen so as to fulfil the condition that the molecular weight ranges from 800 to 2000;
(d) $R'f\ (CF_2CF_2O)_n\ R'f$
wherein n is an integer chosen so as to fulfil the above mentioned condition and R′f is $CF_3$ or $C_2F_5$;
(e) $A(CF_2CF_2CF_2O)_n\ B$
wherein n is an integer chosen so as to fulfil the above conditions, A is F or OR′f and B is R′f or $C_3F_7$;
(f) perfluoropolyethers containing repeating units ($C_2F_4O$), ($C_3F_6O$), (CFXO) and having a molecular weight of from 800 to 2000;
(g)

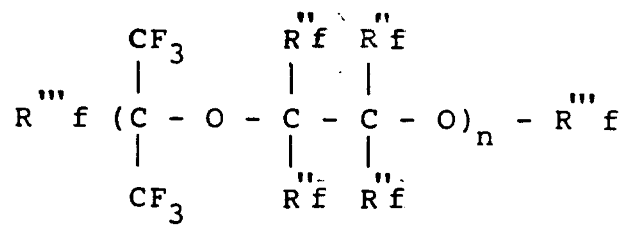

wherein R″f is a perfluoroalkyl group or F, n is an integer chosen so as to fulfil the above condition and R‴f is a perfluoroalkyl group.

**5.** The use of the compound according to claim 1 for carrying out the Gross leak test according to method NID.

**Patentansprüche**

**1.** Perfluor-2,3,4-trimethylpentan.

**2.** Verfahren zur Herstellung der Verbindung nach Anspruch 1, worin die Verbindung der Formel (II):

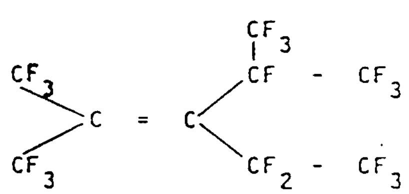

(II)

wahlweise in Anwesenheit einer Verbindung der Formel (III)

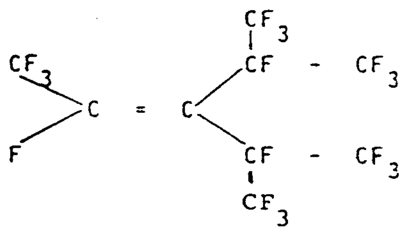

(III)

der Fluorierung mit elementarem Fluor in Gegenwart von UV-Strahlung bei Temperaturen über -40° C, jedoch nicht über 40° C, unterworfen wird.

**3.** Verfahren nach Anspruch 2, worin die Temperatur im Bereich von 10 bis 35°C liegt.

**4.** Verfahren nach Anspruch 2, das man in Anwesenheit eines fluorierten inerten Lösungsmittels durchführt, wobei das Lösungsmittel ein Perfluorpolyether mit einem Molekulargewicht im Bereich von 800 bis 2000 ist, ausgewählt aus einer der folgenden Klassen:

(a) $CF_3O\ (C_2F_4O)p\ (CF_2O)q\ -CF_3$

worin p und q ganze Zahlen sind und der Quotient p/q im Bereich von 1 bis 0,5 liegt und die Einheiten $C_2F_4O$ und $CF_2O$ statistisch über die Kette verteilt sind;

(b) $RfO(C_3F_6O)m\ (CFXO)n\ Rf$

worin Rf $CF_3$, $C_2F_5$ oder $C_3F_7$ ist, X F oder $CF_3$ ist, m und n ganze Zahlen sind, die so ausgewählt sind, daß die Bedingung, daß das Molekulargewicht im Bereich von 800 bis 2000 liegt, erfüllt wird;

(c) $C_3F_7O\ (C_3F_6O)x\ C_2F_5$, worin x eine ganze Zahl ist, die so ausgewählt ist, daß die Bedingung, daß das Molekulargewicht im Bereich von 800 bis 2000 liegt, erfüllt wird;

(d) $R'f\ (CF_2CF_2O)n\ R'f$

worin n eine ganze Zahl ist, die so ausgewählt ist, daß die oben genannte Bedingung erfüllt wird, und R'f $CF_3$ oder $C_2F_5$ ist;

(e) $A(CF_2CF_2CF_2O)_n\ B$

worin n eine ganze Zahl ist, die so ausgewählt ist, daß die oben genannte Bedingung erfüllt wird, A F oder OR'f ist und B R'f oder $C_3F_7$ ist;

(f) Perfluorpolyether, die Struktureinheiten ($C_2F_4O$), ($C_3F_6O$), (CFXO) enthalten und ein Molekulargewicht von 800 bis 2000 haben;

(g)

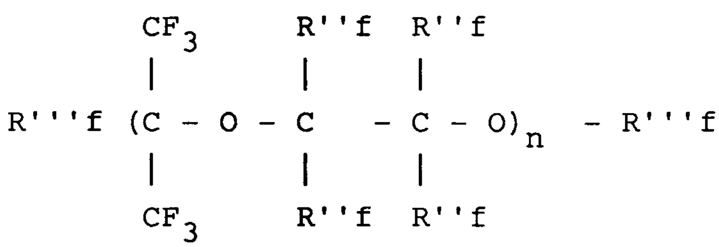

worin R''f eine Perfluoralkylgruppe oder F ist, n eine ganze Zahl ist, die so ausgewählt ist, daß die oben genannte Bedingung erfüllt wird, und R'''f eine Perfluoralkylgruppe ist.

**5.** Verwendung der Verbindung nach Anspruch 1 zur Durchführung des Gross Leak Tests gemäß der NID Methode.

**Revendications**

**1.** Perfluoro-2,3,4-triméthylpentane.

**2.** Un procédé de préparation du composé selon la revendication 1, dans lequel le composé de formule (II):

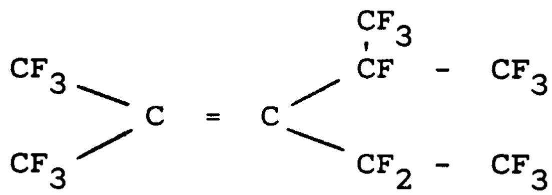 (II)

éventuellement en présence d'un composé de formule (III):

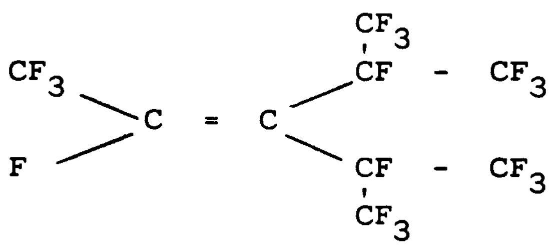 (III)

est soumis à une fluoration avec du fluor élémentaire en présence d'un rayonnement ultraviolet à des températures supérieures à -40 ° C mais ne dépassant 40 ° C.

**3.** Le procédé selon la revendication 2, dans lequel le domaine de température est compris entre 10 et 35 ° C.

**4.** Le procédé selon la revendication 2, mis en oeuvre en présence d'un solvant fluoré inerte, lequel solvant est un perfluoropolyéther présentant un poids moléculaire compris entre 800 et 2000 et qui est choisi dans l'une des classes suivantes:

(a) $CF_3O(C_2F_4O)_p(CF_2O)_q-CF_3$
dans laquelle p et q sont des nombres entiers et le rapport p/q est compris entre 1 et 0,5, les unités $C_2F_4O$ et $CF_2O$ étant réparties de façon statistique le long de la chaîne;

(b) $RfO(C_3F_6)_m(CFXO)_nRf$
dans laquelle Rf est $CF_3$, $C_2F_5$ ou $C_3F_7$, X est soit F, soit $CF_3$, m et n sont des nombres entiers choisis de façon à satisfaire à la condition que le poids moléculaire soit compris entre 800 et 2000;

(c) $C_3F_7O(C_3F_6O)_xC_2F_5$
dans laquelle x est un nombre entier choisi de façon à satisfaire la condition que le poids moléculaire soit compris entre 800 et 2000;

(d) $R'f(CF_2CF_2O)_nR'f$
dans laquelle n est un nombre entier choisi de façon à satisfaire à la condition précitée et que R'f soit $CF_3$ ou $C_2F_5$;

(e) $A(CF_2CF_2CF_2O)_nB$
dans laquelle n est un nombre entier choisi de façon à satisfaire à la condition précitée, A est F ou OR'f et B est R'f ou $C_3F_7$,

(f) les perfluoropolyéthers contenant des motifs répétitifs $(C_2F_4O)$, $(C_3F_6O)$, (CFXO) et présentant un poids moléculaire compris entre 800 et 2000;

(g)

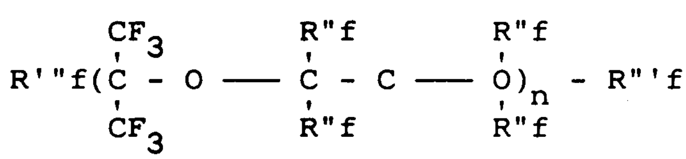

dans laquelle R"'f est un groupe perfluoroalkyle, n est un nombre entier choisi de façon à satisfaire la condition précitée et R"f est un atome de fluor ou un groupe perfluoroalkyle.

5. Application du composé selon la revendication 1, pour la mise en oeuvre du test dénommé Gross leak selon la méthode NID.